# FASCICULE DE BREVET EUROPEEN

(11) **EP 4 338 126 B1**
(45) Date de publication et mention de la délivrance du brevet: **31.12.2025**
(21) Numéro de dépôt: 22724825.9
(22) Date de dépôt: 06.05.2022
(51) Int. Cl.: G06T 7/11

(54) **MÉTHODE DE GÉNÉRATION D'IMAGES MÉDICALES RARES POUR L'ENTRAINEMENT D'ALGORITHMES D'APPRENTISSAGE PROFOND**
VERFAHREN ZUR ERZEUGUNG SELTENER MEDIZINISCHER BILDER ZUM TRAINIEREN VON TIEFENLERNALGORITHMEN
METHOD FOR GENERATING RARE MEDICAL IMAGES FOR TRAINING DEEP-LEARNING ALGORITHMS

(30) Priorité: 11.05.2021 FR 2104970
(43) Date de publication de la demande: 20.03.2024
(73) Titulaire: Quantum Surgical, 34000 Montpellier (FR)
(72) Inventeur: GIRARDOT, Michael, 34170 Castelnau le Lez (FR); BLONDEL, Lucien, 34070 Montpellier (FR); NAHUM, Bertin, 34170 Castelnau le Lez (FR); BADANO, Fernand, 69006 Lyon (FR)
(74) Mandataire: Santarelli
(86) Numéro de dépôt international: PCT/FR2022/050869
(87) Numéro de publication internationale: WO 2022/238640

(56) Documents cités:
- WO-A1-2019/241155
- SHIN HOO-CHANG ET AL: "Medical Image Synthesis for Data Augmentation and Anonymization Using Generative Adversarial Networks", 12 September 2018, ICIAP: INTERNATIONAL CONFERENCE ON IMAGE ANALYSIS AND PROCESSING, 17TH INTERNATIONAL CONFERENCE, NAPLES, ITALY, SEPTEMBER 9-13, 2013. PROCEEDINGS; [LECTURE NOTES IN COMPUTER SCIENCE; LECT.NOTES COMPUTER], SPRINGER, BERLIN, HEIDELBERG, PAGE(S) 1 - 11, ISBN: 978-3-642-17318-9, XP047485123
- CHANGHEE HAN ET AL: "Learning More with Less: Conditional PGGAN-based Data Augmentation for Brain Metastases Detection Using Highly-Rough Annotation on MR Images", ARXIV.ORG, CORNELL UNIVERSITY LIBRARY, 201 OLIN LIBRARY CORNELL UNIVERSITY ITHACA, NY 14853, 26 February 2019 (2019-02-26), XP081467262, DOI: 10.1145/3357384.3357890
- KUGELMAN J ET AL: "Dual image and mask synthesis with GANs for semantic segmentation in optical coherence tomography", PROCEEDINGS OF THE 2020 DIGITAL IMAGE COMPUTING: TECHNIQUES AND APPLICATIONS (DICTA) IEEE PISCATAWAY, NJ, USA, 29 November 2020 (2020-11-29) - 2 December 2020 (2020-12-02), pages 1 - 8, XP002804910, ISBN: 978-1-7281-9108-9
- KARKI M ET AL: "Lesion Conditional Image Generation for Improved Segmentation of Intracranial Hemorrhage from CT Images [arXiv]", 30 March 2020 (2020-03-30), pages 1 - 14, XP002804911, Retrieved from the Internet <URL:http://arxiv.org/abs/2003.13868> [retrieved on 20211129]
- WU WENSHAN ET AL: "Deep Learning for Neuroimaging Segmentation with a Novel Data Augmentation Strategy", 2020 42ND ANNUAL INTERNATIONAL CONFERENCE OF THE IEEE ENGINEERING IN MEDICINE & BIOLOGY SOCIETY (EMBC), IEEE, 20 July 2020 (2020-07-20), pages 1516 - 1519, XP033816522, DOI: 10.1109/EMBC44109.2020.9176537

## Description

### Domaine de l'invention

La présente demande appartient au domaine de la génération d'images médicales synthétiques présentant des anomalies anatomiques rares à l'aide d'un réseau de neurones artificiels. Les images synthétiques générées sont destinées à être utilisées pour entrainer un algorithme d'apprentissage automatique visant à détecter ou caractériser une anomalie au sein d'une anatomie d'intérêt visible sur une image médicale, par exemple un réseau de neurones profonds pour la classification ou la segmentation d'une anomalie.

### Etat de la technique

L'entrainement d'un algorithme d'apprentissage automatique, et plus particulièrement l'entrainement d'un réseau de neurones artificiels, nécessite une grande quantité de données dans le jeu d'entrainement pour atteindre une bonne qualité de prédiction. La sous-représentation d'une classe rare de données dans le jeu d'entrainement a en effet un impact sur les biais de prédiction observés pour une classe plutôt qu'une autre.

Ce problème prend une importance particulière dans le domaine médical, par exemple pour entrainer un réseau de neurones profonds visant à détecter ou caractériser une anomalie au sein d'une anatomie d'intérêt visible sur une image médicale. En effet, il est difficile de collecter des grandes quantités d'images médicales en raison de la rareté des maladies, de la confidentialité des patients, des efforts et des dépenses nécessaires pour mener des opérations d'imagerie médicale, etc.

Plusieurs solutions existantes visent à augmenter artificiellement la quantité de données correspondant à des classes rares dans le jeu d'entrainement en créant des images médicales synthétiques présentant des anomalies anatomiques rares. Toutefois, dans les solutions de l'art antérieur, les images synthétiques obtenues ne sont généralement pas suffisamment différentes des images réelles à partir desquelles elles sont générées, et elles ne permettent pas d'entrainer de façon optimale un réseau de neurones profonds de classification ou de segmentation. Il y a alors en effet un risque de sur-apprentissage sur une classe particulière au détriment d'autres classes. Le sur-apprentissage désigne le phénomène de perte de généralisation des prédictions d'un algorithme : les prédictions sont bonnes sur les données du jeu d'entrainement mais elles sont mauvaises sur des données nouvelles.

La demande de brevet US 2019/0370969 A1 enseigne par exemple d'entrainer un algorithme de classification d'une tumeur avec des images synthétiques générées par des réseaux de neurones antagonistes génératifs (« Generative Adversial Network » ou GAN dans la littérature anglo-saxonne). L'algorithme de classification est entrainé avec des images synthétiques mélangées avec des images réelles. Plusieurs réseaux de neurones antagonistes génératifs sont nécessaires pour produire des images de tumeurs de différentes classes. La méthode d'entrainement de l'algorithme de classification comprend une méthode d'ajustement du poids des images en fonction de la performance d'apprentissage de l'algorithme, ce qui permet de renforcer l'apprentissage des cas difficiles et en particulier les cas sous représentés dans le jeu d'entrainement. L'inconvénient majeur de cette méthode est la nécessité d'entrainer plusieurs réseaux de neurones antagonistes génératifs pour produire différentes classes d'images synthétiques, ce qui est techniquement difficile. Une autre limitation de cette approche est que la diversité des images synthétiques obtenues est limitée par la diversité des images utilisées pour l'entrainement des réseaux de neurones antagonistes génératifs. Les images synthétiques de classes rares sont difficiles à produire si elles ne sont pas initialement présentes dans les jeux de données d'entrainement des réseaux de neurones antagonistes génératifs.

Certaines solutions mettent en œuvre au moins deux générateurs de réseaux de neurones antagonistes génératifs interdépendants l'un avec l'autre. Une telle approche augmente significativement la complexité technique, notamment parce que l'optimisation de l'un des générateurs dépend de l'optimisation de l'autre.

Il existe donc toujours un besoin d'une solution relativement simple à mettre en œuvre pour créer un grand nombre et une grande variété d'images d'anomalies anatomiques rares pour entrainer un algorithme d'apprentissage visant à détecter ou caractériser une anomalie.

Le document « Medical Image Synthesis for Data Augmentation and Anonymization Using Generative Adversial Networks », Shin Hoo-Chang et Al., décrit une méthode pour générer des images médicales synthétiques avec un réseau de neurones de type GAN.

### Exposé de l'invention

Les méthodes et les dispositifs divulgués dans la présente demande ont pour objectif de remédier à tout ou partie des inconvénients de l'art antérieur, notamment ceux exposés ci-avant.

A cet effet, et selon un premier aspect, il est proposé une méthode selon la revendication 1 pour générer des images médicales synthétiques représentant une anatomie d'intérêt et une anomalie au sein de ladite anatomie d'intérêt.

L'anatomie d'intérêt peut correspondre à un organe (par exemple le foie, le pancréas, la vésicule biliaire, un poumon ou un rein) ou à une autre structure anatomique (par exemple un os ou un vaisseau sanguin). Une anomalie présente au sein de l'anatomie d'intérêt correspond généralement à une lésion, comme par exemple une tumeur, un kyste, une zone d'ablation, un anévrisme, etc. Une zone d'ablation correspond à une lésion ayant subi un traitement d'ablation selon une méthode connue (micro-onde, laser, radiofréquence, etc.). Il s'agit d'une région nécrosée.

On entend par « image médicale réelle » une image médicale acquise sur un patient à l'aide d'un dispositif d'imagerie médicale, par exemple par tomodensitométrie (« CT scan » dans la littérature anglo-saxonne, CT est l'acronyme de « computerized tomography »), par tomographie par émission de positons (« PET scan » dans la littérature anglo-saxonne, PET est l'acronyme de « positron emission tomography »), par imagerie par résonance magnétique (IRM), par ultrasons, ou par rayons X.

Les termes « majoritaires » et « minoritaires » sont utilisés car il y a généralement un nombre significativement plus grand d'images médicales représentant une anatomie d'intérêt sans anomalie que d'images médicales représentant une anatomie d'intérêt avec une anomalie.

Une image médicale « synthétique » est en revanche générée artificiellement par le réseau de neurones. Un réseau de neurones est un système informatique matériel et/ou logiciel dont le fonctionnement est inspiré de celui des neurones du cerveau humain. Il s'agit d'une variété de technologie d'apprentissage profond (« deep learning » dans la littérature anglo-saxonne), qui fait elle-même partie des algorithmes d'apprentissage automatique. Les algorithmes d'apprentissage automatique forment une catégorie du domaine de l'intelligence artificielle.

Le réseau de neurones est entrainé pour générer une image synthétique à partir d'un masque de segmentation associé à une image médicale réelle. Les masques de segmentation majoritaires et les masques de segmentation minoritaires peuvent être utilisés pour entrainer le réseau de neurones. Un masque de segmentation est une image dont chaque voxel fournit une information particulière sur un élément représenté à la position du voxel sur l'image médicale réelle. Un voxel peut ainsi prendre une valeur numérique particulière associée à l'élément représenté à la position du voxel sur l'image médicale réelle (une valeur numérique spécifique est par exemple définie pour une partie saine de l'anatomie d'intérêt, et une valeur numérique différente est définie pour une anomalie). Il convient de noter que le terme « voxel » est utilisé de façon générique pour définir une zone particulière d'une image (un voxel identifie une position de ladite zone sur l'image et prend une valeur représentative de ce qui est représenté dans ladite zone sur l'image). Il peut s'agir d'une image en deux dimensions ou en trois dimensions. S'il s'agit d'une image en deux dimensions, le terme « voxel » prend alors la même signification que le terme « pixel ».

Différents types de réseaux de neurones peuvent être envisagés pour générer une image synthétique à partir d'un masque de segmentation. Par exemple, il est envisageable d'utiliser un réseau de neurones de type auto-encodeur (« Variational Autoencoder » ou VAE dans la littérature anglo-saxonne). Il est toutefois préférable d'utiliser un générateur d'un couple de réseaux de neurones antagonistes génératifs (GAN). Ces types de réseaux de neurones permettent en effet de générer des images avec un fort degré de réalisme. Un GAN est un modèle génératif où deux réseaux de neurones sont placés en compétition dans un scénario de jeu à somme nulle. Le premier réseau, le générateur, génère une image, et son adversaire, le discriminateur, essaye de détecter si l'image générée est réelle ou bien s'il s'agit d'une image synthétique générée par le générateur.

Il est avantageux de générer une image synthétique à partir d'un masque de segmentation, et non pas à partir de « bruit » aléatoire comme c'est généralement le cas dans l'art antérieur. L'utilisation d'un masque de segmentation en entrée du réseau de neurones permet d'avoir plus de contrôle sur l'image synthétique produite par le réseau de neurones.

L'invention repose sur la génération de masques de segmentation artificiels à partir d'un ensemble de masques de segmentation majoritaires associés à des images médicales réelles majoritaires et d'un ensemble de masques de segmentation minoritaires associés à des images médicales réelles minoritaires. Pour générer un masque de segmentation artificiel, la segmentation de l'anatomie d'intérêt d'un masque de segmentation majoritaire est combinée avec la segmentation de l'anomalie d'un masque de segmentation minoritaire. La segmentation de l'anomalie est par exemple intégrée dans le masque de segmentation majoritaire à différentes positions dans l'anatomie d'intérêt, et selon différentes orientations. Il est également envisageable de déformer la segmentation de l'anomalie d'intérêt avant de l'intégrer dans le masque de segmentation majoritaire. Chaque masque de segmentation majoritaire peut être combiné avec chaque masque de segmentation minoritaire.

De telles dispositions permettent de générer une très grande diversité de masques de segmentation artificiels. Cela permet alors ensuite de générer une très grande diversité d'images médicales synthétiques présentant des anomalies anatomiques.

Cette diversité des images médicales synthétiques permet de générer un jeu d'images d'entrainement particulièrement efficace pour un algorithme d'apprentissage automatique visant à détecter ou caractériser une anomalie dans l'anatomie d'intérêt d'un patient sur une image médicale réelle. Le jeu d'images d'entrainement peut comprendre à la fois des images médicales réelles et des images médicales synthétiques. Le nombre d'images médicales présentant une anomalie et la variété des anomalies sont considérablement augmentés grâce à la grande variété des masques de segmentation artificiels. De préférence, le jeu d'images d'entrainement comprend un nombre d'images avec anomalie sensiblement égal au nombre d'images sans anomalie. Cela permet ainsi d'éviter le phénomène de sur-apprentissage sur une classe particulière d'anomalie.

En outre, la complexité technique de la solution proposée est relativement limitée. En particulier, la génération des masques de segmentation artificiels ne nécessite pas l'utilisation d'un algorithme d'apprentissage. La méthode proposée pour générer des images médicales synthétiques nécessite donc au plus un seul GAN (dans l'hypothèse où le réseau de neurones utilisé pour générer les images synthétiques est un GAN).

Il convient également de noter que la méthode proposée n'est pas limitée à la génération d'images médicales synthétiques représentant une seule anatomie d'intérêt avec une seule anomalie au sein de ladite anatomie d'intérêt. Autrement dit, la méthode peut aussi permettre de générer des images médicales synthétiques représentant une anatomie d'intérêt avec plusieurs anomalies au sein de l'anatomie d'intérêt (les anomalies pouvant éventuellement être de différentes natures). Aussi, la méthode peut permettre de générer des images médicales synthétiques représentant plusieurs anatomies d'intérêt différentes avec éventuellement plusieurs anomalies au sein de chaque anatomie d'intérêt représentée. Dans ce but, les masques de segmentation artificiels peuvent être générés à partir de combinaisons de masques de segmentation majoritaires correspondant à des anatomies d'intérêt différentes et de masques de segmentation minoritaires correspondant à des types d'anomalies différents.

Dans des modes particuliers de mise en œuvre, la méthode peut comporter en outre l'une ou plusieurs des caractéristiques suivantes, prises isolément ou selon toutes les combinaisons techniquement possibles.

Dans des modes particuliers de mise en œuvre, la génération d'un masque de segmentation artificiel comporte en outre une transformation de la segmentation de l'anomalie du masque de segmentation minoritaire.

Dans des modes particuliers de mise en œuvre, la transformation de la segmentation de l'anomalie correspond à une rotation, un agrandissement, une réduction, une déformation et/ou un déplacement de la segmentation de l'anomalie.

Dans des modes particuliers de mise en œuvre, la génération d'un masque de segmentation artificiel comporte en outre une vérification que la segmentation de l'anomalie par rapport à la segmentation de l'anatomie d'intérêt satisfait un critère particulier.

Il s'agit par exemple de vérifier que certaines contraintes particulières sont satisfaites afin de filtrer les combinaisons non réalistes. Ces contraintes sont par exemple relatives à la localisation de l'anomalie au sein de l'organe d'intérêt ou par rapport à d'autres organes ou d'autres structures anatomiques.

Dans des modes particuliers de mise en œuvre, un masque de segmentation comporte un ensemble de voxels, chaque voxel correspondant à une zone de l'image médicale réelle à laquelle le masque de segmentation est associé, chaque voxel étant associé à une valeur numérique encodant ce qui est représenté par ladite zone sur l'image médicale réelle, et l'étape de génération d'un masque de segmentation artificiel comporte :
- une sélection d'un masque de segmentation majoritaire et d'un masque de segmentation minoritaire,
- une identification, sur le masque de segmentation minoritaire sélectionné, d'un ensemble de voxels dont la valeur numérique encode l'anomalie,
- un remplacement, sur le masque de segmentation majoritaire sélectionné, de la valeur numérique des voxels identifiés par la valeur numérique encodant l'anomalie.

Dans des modes particuliers de mise en œuvre, le réseau de neurones utilisé pour générer une image médicale synthétique est un réseau de neurones générateur, et l'entrainement du réseau de neurones générateur est mise en œuvre à l'aide d'un réseau de neurones discriminateur, le réseau de neurones générateur et le réseau de neurones discriminateur formant un couple de réseaux antagonistes génératifs.

Il est généralement difficile d'obtenir des images de haute résolution avec des réseaux de neurones antagonistes génératifs (GANs). Pour pallier cet inconvénient, la méthode selon l'invention peut avantageusement comporter une étape préalable de réduction de la taille des images médicales réelles majoritaires et minoritaires. Par exemple, les images médicales peuvent être réduites à une taille de 128x128 pixels (s'il s'agit d'une image en deux dimensions) et centrées sur l'anatomie d'intérêt.

Dans des modes particuliers de mise en œuvre, les images médicales réelles à partir desquelles sont générés les masques de segmentation majoritaires et les masques de segmentation minoritaires sont des images médicales obtenues par tomodensitométrie, par tomographie par émission de positons, par imagerie par résonance magnétique ou par ultrasons.

Dans des modes particuliers de mise en œuvre, l'anatomie d'intérêt est un organe tel que le foie, un poumon ou un rein, ou une autre structure anatomique telle qu'un os ou un vaisseau sanguin.

Dans des modes particuliers de mise en œuvre, l'anomalie est une tumeur ou une zone d'ablation.

Selon un deuxième aspect, il est proposé une méthode pour entrainer un algorithme d'apprentissage automatique visant à détecter ou caractériser une anomalie dans l'anatomie d'intérêt d'un patient sur une image médicale réelle. La méthode comprend une génération, à l'aide d'une méthode selon l'un quelconque des modes de mise en œuvre précédents, d'images médicales synthétiques représentant l'anatomie d'intérêt et une anomalie au sein de ladite anatomie intérêt. La méthode comprend ensuite un entrainement de l'algorithme d'apprentissage automatique avec un ensemble d'images d'entrainement comportant les images médicales synthétiques ainsi générées.

Dans des modes particuliers de mise en œuvre, la méthode peut comporter en outre l'une ou plusieurs des caractéristiques suivantes, prises isolément ou selon toutes les combinaisons techniquement possibles.

Dans des modes particuliers de mise en œuvre, l'ensemble d'images d'entrainement comporte des images médicales synthétiques et des images médicales réelles, et le nombre d'images avec une anomalie est au moins égal à 10% du nombre d'images sans anomalie.

Dans des modes particuliers de mise en œuvre, l'algorithme d'apprentissage automatique est un algorithme de classification de l'anomalie.

Dans des modes particuliers de mise en œuvre, l'algorithme d'apprentissage automatique est un algorithme de segmentation de l'anomalie.

Dans des modes particuliers de mise en œuvre, l'algorithme d'apprentissage automatique est un mis en œuvre par un réseau de neurones profonds.

Selon un troisième aspect, il est proposé une méthode pour détecter ou caractériser une anomalie dans l'anatomie d'intérêt d'un patient sur une image médicale réelle. La méthode comprend :
- un entrainement, avec une méthode selon l'un quelconque des modes de mise en œuvre précédents, d'un algorithme d'apprentissage automatique visant à détecter ou caractériser une anomalie dans l'anatomie d'intérêt d'un patient sur une image médicale réelle,
- une réception d'une image médicale réelle de l'anatomie d'intérêt d'un patient,
- une analyse de ladite image médicale réelle avec l'algorithme d'apprentissage automatique entrainé,
- une obtention, en sortie de l'algorithme d'apprentissage automatique entrainé, d'une information permettant de détecter ou de caractériser une anomalie dans l'anatomie d'intérêt visible sur l'image médicale réelle.

Selon un quatrième aspect, il est proposé un dispositif selon la revendication 14.

Dans des modes particuliers de réalisation, le dispositif peut comporter en outre l'une ou plusieurs des caractéristiques suivantes, prises isolément ou selon toutes les combinaisons techniquement possibles.

Dans des modes particuliers de réalisation, pour générer un masque de segmentation artificiel, le ou les processeurs sont en outre configurés pour transformer la segmentation de l'anomalie du masque de segmentation minoritaire.

Dans des modes particuliers de réalisation, la transformation de la segmentation de l'anomalie correspond à une rotation, un agrandissement, une réduction, une déformation ou un déplacement de la segmentation de l'anomalie.

Dans des modes particuliers de réalisation, pour générer un masque de segmentation artificiel, le ou les processeurs sont en outre configurés pour vérifier que la segmentation de l'anomalie par rapport à la segmentation de l'anatomie d'intérêt satisfait un critère particulier.

Dans des modes particuliers de réalisation, un masque de segmentation comporte un ensemble de voxels. Chaque voxel correspond à une zone de l'image médicale réelle à laquelle le masque de segmentation est associé. Chaque voxel est associé à une valeur numérique encodant ce qui est représenté par ladite zone sur l'image médicale réelle. Pour générer un masque de segmentation artificiel, le ou les processeurs sont configurés pour :
- sélectionner un masque de segmentation majoritaire et un masque de segmentation minoritaire,
- identifier, sur le masque de segmentation minoritaire sélectionné, un ensemble de voxels dont la valeur numérique encode l'anomalie,
- remplacer, sur le masque de segmentation majoritaire sélectionné, la valeur numérique des voxels identifiés par la valeur numérique encodant l'anomalie.

Dans des modes particuliers de réalisation, le support de stockage mémorise en outre un réseau de neurones préalablement entrainé à générer une image médicale synthétique à partir d'un masque de segmentation et, lorsque le programme est exécuté, le ou les processeurs sont configurés pour générer des images médicales synthétiques avec le réseau de neurones à partir de masques de segmentation artificiels.

Dans des modes particuliers de réalisation, le réseau de neurones pour générer une image médicale synthétique est un réseau de neurones générateur adapté pour être entrainé à l'aide d'un réseau de neurones discriminateur, et le couple formé par le réseau de neurones générateur et le réseau de neurones discriminateur forme un couple de réseaux antagonistes génératifs.

### Présentation des figures

L'invention sera mieux comprise à la lecture de la description suivante, donnée à titre d'exemple nullement limitatif, et faite en se référant aux figures 1 à 14 qui représentent :
[Fig. 1] une représentation schématique des principales étapes d'une méthode selon l'invention pour générer des images médicales synthétiques représentant une anomalie anatomique,
[Fig. 2] une représentation schématique des principales étapes d'une méthode selon l'invention pour entrainer un algorithme d'apprentissage automatique visant à détecter ou caractériser une anomalie dans l'anatomie d'intérêt d'un patient sur une image médicale réelle,
[Fig. 3] une représentation schématique des principales étapes d'une méthode selon l'invention pour détecter ou caractériser une anomalie dans l'anatomie d'intérêt d'un patient sur une image médicale réelle,
[Fig. 4] une représentation schématique d'une étape de génération de masques de segmentation majoritaires à partir d'images médicales réelles majoritaires,
[Fig. 5] une représentation schématique d'une étape de génération de masques de segmentation minoritaires à partir d'images médicales réelles minoritaires,
[Fig. 6] une représentation schématique d'une étape de génération de masques de segmentation artificiels à partir de masques de segmentation majoritaires et de masques de segmentation minoritaires,
[Fig. 7] une représentation schématique d'une étape de génération de masques de segmentation artificiels comprenant une transformation de la segmentation de l'anomalie,
[Fig. 8] une illustration de la génération d'un masque de segmentation artificiel à partir d'un masque de segmentation majoritaire et d'un masque de segmentation minoritaire,
[Fig. 9] une représentation schématique d'une étape d'entrainement d'un réseau de neurones à générer une image médicale synthétique à partir d'un masque de segmentation,
[Fig. 10] une illustration d'une image médicale réelle, de son masque de segmentation associé, et d'une image médicale synthétique générée par le réseau de neurones à partir du masque de segmentation, pour un cas majoritaire (sans anomalie) et un cas minoritaire (avec anomalie),
[Fig. 11] une représentation schématique d'une étape de génération d'une image médicale synthétique à partir d'un masque de segmentation artificiel à l'aide du réseau de neurones préalablement entrainé,
[Fig. 12] une illustration, pour trois images médicales réelles majoritaires différentes, de l'image médicale réelle majoritaire, de son masque de segmentation majoritaire associé, d'un masque de segmentation artificiel généré à partir du masque de segmentation majoritaire et d'un masque de segmentation minoritaire, et d'une image médicale synthétique générée par le réseau de neurones à partir du masque de segmentation artificiel,
[Fig. 13] une représentation schématique d'une étape d'entrainement, à partir d'une image médicale synthétique, de l'algorithme d'apprentissage automatique,
[Fig. 14] une représentation schématique de la détection ou de la caractérisation d'une anomalie dans une anatomie d'intérêt représentée sur une image médicale réelle par l'algorithme d'apprentissage automatique.

Dans ces figures, des références identiques d'une figure à une autre désignent des éléments identiques ou analogues. Pour des raisons de clarté, les éléments représentés ne sont pas nécessairement à une même échelle, sauf mention contraire.

### Description détaillée d'au moins un mode de réalisation de l'invention

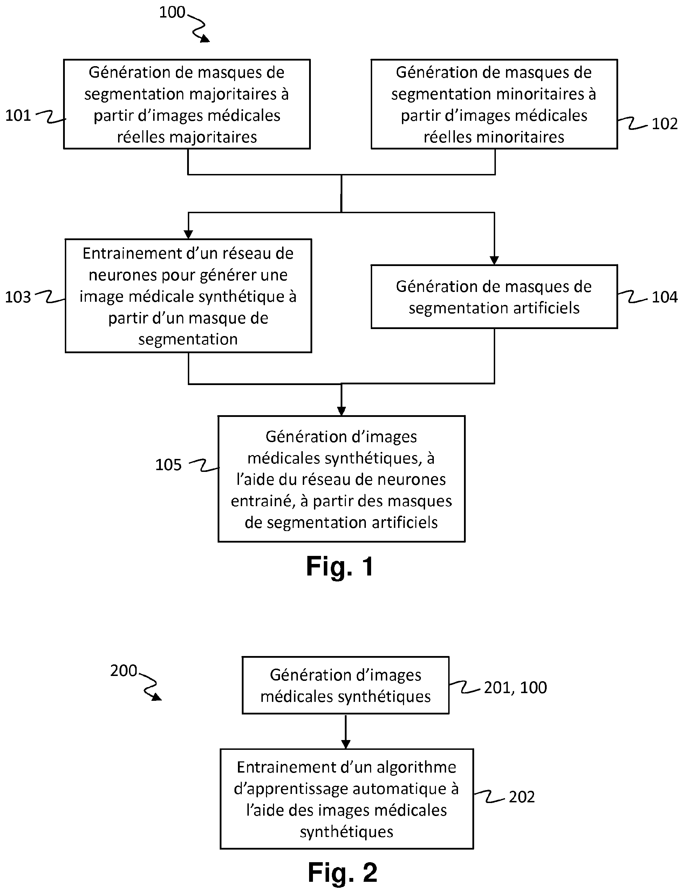

La figure 1 représente schématiquement les principales étapes d'une méthode 100 selon l'invention pour générer des images médicales synthétiques représentant une anomalie dans une anatomie d'intérêt.

Dans la suite de la description, on considère à titre nullement limitatif que l'anatomie d'intérêt est le foie et que l'anomalie est une tumeur. Il convient toutefois de noter que la méthode pourrait s'appliquer à d'autres anatomies d'intérêt comme par exemple un poumon, un rein, un os, un vaisseau sanguin, etc. Aussi, la méthode pourrait s'appliquer à d'autres types d'anomalies, comme par exemple une tumeur, un kyste, une zone d'ablation, un anévrisme, etc.

La méthode 100 comprend une étape de génération 101 de masques de segmentation majoritaires. Chaque masque de segmentation majoritaire est associé à une image médicale réelle majoritaire représentant l'anatomie d'intérêt d'un patient dans un cas où ladite anatomie d'intérêt ne présente pas d'anomalie.

Dans la suite de la description, on considère qu'une image médicale réelle est une image en deux dimensions acquises par un dispositif d'imagerie par tomodensitométrie. Rien n'empêcherait toutefois, dans des variantes, d'utiliser des images médicales réelles en trois dimensions ou des images médicales réelles acquises selon d'autres modalités d'imagerie, comme par exemple par résonance magnétique, par tomographie par émission de positons, par ultrasons, ou par rayons X.

La méthode 100 comprend également une étape de génération 102 de masques de segmentation minoritaires. Chaque masque de segmentation minoritaire est associé à une image médicale réelle minoritaire représentant l'anatomie d'intérêt d'un patient dans un cas où ladite anatomie d'intérêt présente une anomalie.

Il convient de noter que l'ordre des étapes 101 et 102 n'a pas d'importance. D'autre part, la méthode 100 peut optionnellement comporter une étape préalable de réduction de la taille des images médicales réelles. Par exemple, les images médicales peuvent être réduites à une taille de 128x128 pixels (s'il s'agit d'une image en deux dimensions) et centrées sur l'anatomie d'intérêt, avant d'être utilisées pour générer les masques de segmentation.

La méthode 100 comprend une étape d'entrainement 103 d'un réseau de neurones 31 pour générer une image médicale synthétique à partir d'un masque de segmentation. Pour entraîner le réseau de neurones, on peut utiliser par exemple les masques de segmentation majoritaire générés à l'étape 101 et/ou les masques de segmentation minoritaire générés à l'étape 102. D'autres masques de segmentation peuvent cependant aussi être utilisés.

Dans la suite de la description, on considère que le réseau de neurones utilisé pour générer une image médicale synthétique à partir d'un masque de segmentation est un générateur d'un couple de réseaux de neurones antagonistes génératifs (GAN). Il convient toutefois de noter qu'il serait également possible d'utiliser d'autres types de réseaux de neurones, comme par exemple un réseau de neurones de type auto-encodeur (VAE). Le choix d'un type particulier de réseau de neurones pour générer une image médicale synthétique à partir d'un masque de segmentation n'est qu'une variante de l'invention.

La méthode 100 comprend une étape de génération 104 de masques de segmentation artificiels à partir des masques de segmentation majoritaires et des masques de segmentation minoritaires. La génération 104 d'un masque de segmentation artificiel est basée sur une combinaison d'une segmentation de l'anatomie d'intérêt d'un masque de segmentation majoritaire avec une segmentation de l'anomalie 25 d'un masque de segmentation minoritaire.

Il convient de noter que les étapes 103 et 104 sont indépendantes l'une de l'autre, et que l'ordre dans lequel elles sont exécutées importe peu.

Enfin, la méthode 100 comprend une étape de génération 105 d'images médicales synthétiques à partir des masques de segmentation artificiels à l'aide du réseau de neurones préalablement entrainé.

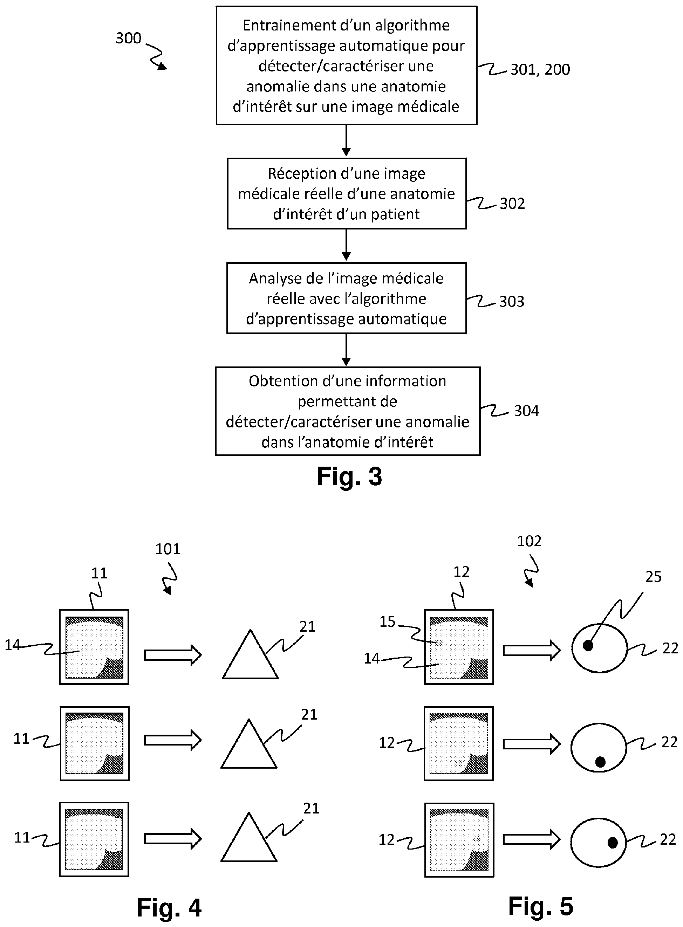

Les étapes 101 à 105 seront détaillées ultérieurement en référence aux figures 4 à 12.

La figure 2 représente schématiquement les principales étapes d'une méthode 200 selon l'invention pour entrainer un algorithme d'apprentissage automatique visant à détecter ou caractériser une anomalie dans l'anatomie d'intérêt d'un patient sur une image médicale réelle.

La méthode 200 comprend notamment une étape de génération 201 d'images médicales synthétiques représentant l'anatomie d'intérêt et une anomalie au sein de ladite anatomie intérêt. La génération 201 d'images médicales synthétiques est mise en œuvre selon la méthode 100 décrite ci-avant en référence à la figure 1.

La méthode 200 comprend ensuite une étape d'entrainement 202 de l'algorithme d'apprentissage automatique avec un ensemble d'images d'entrainement comportant les images médicales synthétiques générées à l'étape 201.

L'algorithme d'apprentissage automatique correspond par exemple à un réseau de neurones profonds. Rien n'empêcherait toutefois d'utiliser d'autres types d'algorithmes d'apprentissage automatique, comme par exemple un algorithme de forêts aléatoires (« random forest » dans la littérature anglo-saxonne).

L'algorithme d'apprentissage automatique correspond par exemple à un algorithme de classification qui permet d'identifier la nature de l'anomalie (type de tumeur). Selon un autre exemple, l'algorithme d'apprentissage automatique correspond à un algorithme de segmentation qui permet de définir le contour de l'anomalie sur une image médicale.

L'ensemble d'images d'entrainement peut comprendre à la fois des images médicales réelles et des images médicales synthétiques. Le nombre d'images médicales présentant une anomalie et la variété des anomalies sont considérablement augmentés grâce à la grande variété des masques de segmentation artificiels utilisés pour générer les images médicales synthétiques. De préférence, le nombre d'images avec une anomalie est au moins égal à 10% du nombre d'images sans anomalie. Idéalement, le jeu d'images d'entrainement comprend un nombre d'images avec anomalie sensiblement égal au nombre d'images sans anomalie. Cela permet ainsi d'éviter le phénomène de sur-apprentissage sur une classe particulière d'anomalie.

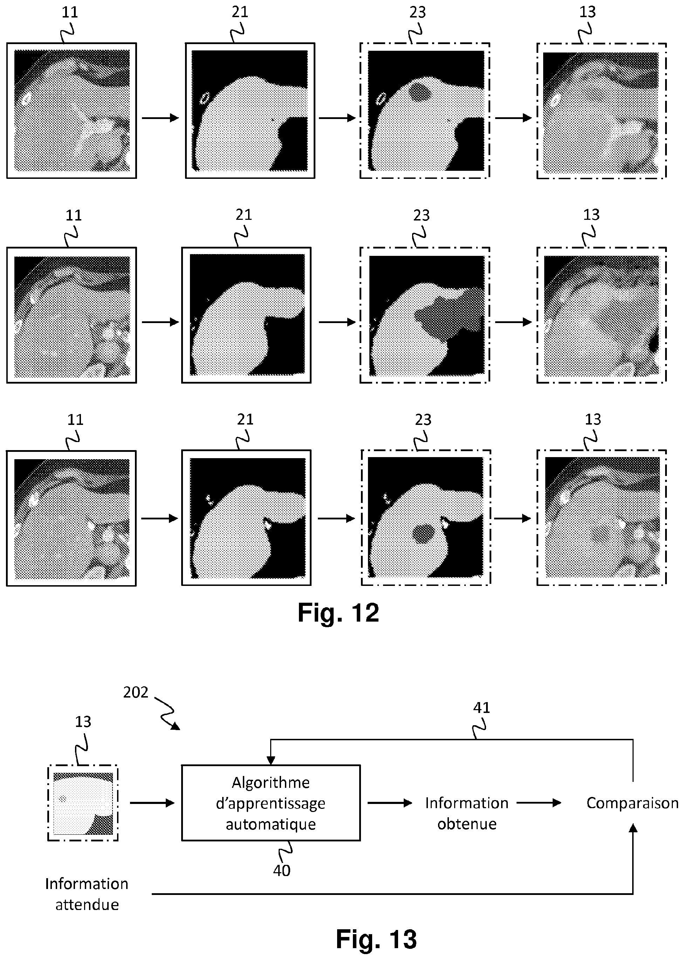

L'étape d'entrainement 202 de l'algorithme d'apprentissage automatique sera détaillé ultérieurement en référence à la figure 13.

La figure 3 représente schématiquement les principales étapes d'une méthode 300 selon l'invention pour détecter ou caractériser une anomalie dans l'anatomie d'intérêt d'un patient sur une image médicale réelle.

La méthode 300 comprend notamment une étape d'entrainement 301 d'un algorithme d'apprentissage automatique visant à détecter ou caractériser une anomalie dans l'anatomie d'intérêt d'un patient sur une image médicale réelle. Cette étape d'entrainement 301 est mise en œuvre selon la méthode 200 décrite ci-avant en référence à la figure 2.

La méthode 300 comprend ensuite successivement une étape de réception 302 d'une image médicale réelle de l'anatomie d'intérêt d'un patient ; une étape d'analyse 303 de ladite image médicale réelle avec l'algorithme d'apprentissage automatique entrainé ; et une étape d'obtention 304, en sortie de l'algorithme d'apprentissage automatique entrainé, d'une information permettant de détecter ou de caractériser une anomalie dans l'anatomie d'intérêt visible sur l'image médicale réelle.

La figure 4 représente schématiquement l'étape de génération 101 d'un masque de segmentation majoritaire 21 à partir d'une image médicale réelle majoritaire 11. L'anatomie d'intérêt 14 est visible sur l'image médicale réelle majoritaire 11. Sur une image médicale réelle dite « majoritaire », il n'y a pas d'anomalie au sein de l'anatomie d'intérêt. Le masque de segmentation majoritaire 21 comporte une segmentation de l'anatomie d'intérêt.

La figure 5 représente schématiquement l'étape de génération 102 d'un masque de segmentation minoritaire 22 à partir d'une image médicale réelle minoritaire 12. L'anatomie d'intérêt 14 est visible sur l'image médicale réelle minoritaire 12. Sur une image médicale réelle dite « minoritaire », une anomalie 15 est présente au sein de l'anatomie d'intérêt 14. Le masque de segmentation minoritaire 22 comporte une segmentation de l'anatomie d'intérêt et une segmentation de l'anomalie 25.

En imagerie médicale, la segmentation est une étape primordiale qui consiste à extraire, à partir d'une image, une ou plusieurs régions anatomiques particulières. Un masque de segmentation est une image dont chaque voxel (ou pixel dans le cas d'une image en deux dimensions) fournit une information particulière sur un élément représenté à la position du voxel sur l'image médicale réelle. Un voxel peut ainsi prendre une valeur numérique particulière associée à l'élément représenté à la position du voxel sur l'image médicale réelle. Des valeurs numériques spécifiques différentes sont par exemple définies respectivement pour une partie saine de l'anatomie d'intérêt, pour une anomalie au sein de l'anatomie d'intérêt, pour d'autres structures anatomiques (par exemple des os, des vaisseaux sanguins), pour le fond de l'image, etc.

La segmentation peut être mise en œuvre de façon manuelle. Dans ce cas le praticien définit lui-même les contours des différentes régions anatomiques sur une image médicale à l'aide d'une interface graphique (souris, stylet, écran tactile, etc.) d'un dispositif électronique (ordinateur, tablette, etc.) sur lequel l'image est affichée. La segmentation peut également être mise en œuvre de façon automatique, à l'aide d'un algorithme d'intelligence artificielle de segmentation.

Par exemple, les os visibles sur les images médicales sont segmentés par la méthode de seuillage d'intensité des voxels avec une fenêtre d'intensité de largeur 1800 HU et de centre 400 HU (HU est l'acronyme anglais de « Houndsfield Unit », il s'agit d'une échelle quantitative décrivant la radio-densité, c'est-à-dire une unité de mesure représentative de l'opacité d'un matériau à une onde radio).

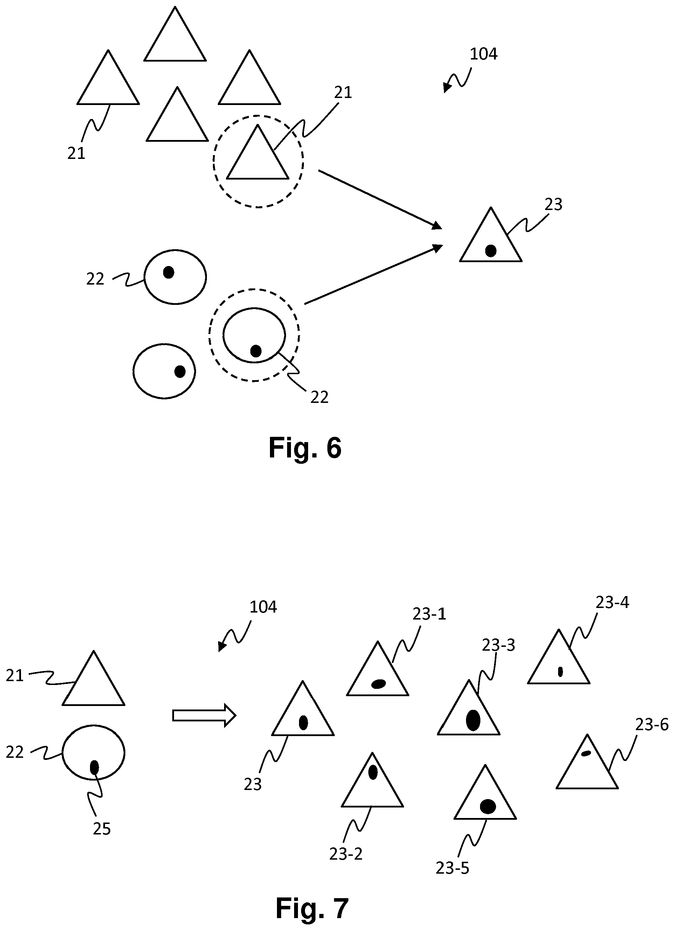

La figure 6 représente schématiquement l'étape de génération 104 de masques de segmentation artificiels 23 à partir de masques de segmentation majoritaires 21 et de masques de segmentation minoritaires 22.

Dans l'exemple illustré à la figure 6, l'étape de génération 104 d'un masque de segmentation artificiel 23 comporte :
- une sélection d'un masque de segmentation majoritaire 21,
- une sélection d'un masque de segmentation minoritaire 22,
- une identification, sur le masque de segmentation minoritaire 22 sélectionné, d'un ensemble de voxels dont la valeur numérique encode l'anomalie,
- un remplacement, sur le masque de segmentation majoritaire 21 sélectionné, de la valeur numérique des voxels identifiés par la valeur numérique encodant l'anomalie.

Le masque de segmentation majoritaire 21 et le masque de segmentation minoritaire 22 peuvent être sélectionnés de façon aléatoire. Le nombre de combinaisons de masques de segmentation artificiels peut ainsi s'élever au produit du nombre de masques de segmentation majoritaires 21 par le nombre de masques de segmentation minoritaires 22.

Plusieurs masques de segmentation minoritaires 21 avec différentes valeurs sémantiques (c'est-à-dire avec différents types d'anomalie : tumeur, kyste, région d'ablation, artefact, ...) peuvent être combinés avec les masques de segmentation majoritaires 22 et ainsi accroitre le nombre de combinaisons potentielles. La manipulation des proportions des différents masques minoritaires 21 qui seront utilisés dans les combinaisons de masques de segmentation artificiels 23 permet de contrôler les caractéristiques des images synthétiques produites. De même, plusieurs masques majoritaires 22 avec différentes valeurs sémantiques (foie, poumon, pancréas, vésicule biliaire, ...) peuvent être combinés avec les masques de segmentation minoritaires 21. Ces différents masques majoritaires 22 permettent de contrôler dans quel organe ou structure peuvent apparaitre les masques de segmentation minoritaires 21 grâce à des règles de logique booléenne (par exemple des règles de type « AND », « OR », « NOT ») entre les différents masques majoritaires 22 et masques minoritaires 21 pour chaque pixel des masques de segmentation artificiels 23. Cette caractéristique permet de contrôler, par exemple, dans quel organe la répartition aléatoire d'un masque de segmentation minoritaire 21 d'une tumeur peut apparaitre (« AND ») et dans quelle structure ou organe ce masque de segmentation minoritaire 21 ne peut pas apparaitre (« NOT »).

Les combinaisons de masque de segmentation majoritaire 21 et de masque de segmentation minoritaire 22 de plus grand intérêt peuvent aussi être sélectionnés en fonction d'une distance estimée entre une segmentation de l'anomalie 25 sur le masque de segmentation minoritaire 22 et une segmentation d'intérêt du masque de segmentation majoritaire 21, comme par exemple la segmentation de la vésicule biliaire, des vaisseaux, du hile ou encore de la capsule du foie.

Tel qu'illustré à la figure 7, l'étape de génération 104 de masques de segmentation artificiels 23 peut comprendre une transformation de la segmentation de l'anomalie 25 du masque de segmentation minoritaire 22 sélectionné. De telles dispositions permettent de générer un plus grand nombre de masques de segmentation artificiels différents. La transformation de la segmentation de l'anomalie 25 correspond par exemple à une rotation (tel qu'illustré pour le masque de segmentation artificiel 23-1), un déplacement (tel qu'illustré pour le masque de segmentation artificiel 23-2), un agrandissement (tel qu'illustré pour le masque de segmentation artificiel 23-3), une réduction (tel qu'illustré pour le masque de segmentation artificiel 23-4), une déformation (tel qu'illustré pour le masque de segmentation artificiel 23-5) ou une combinaison des ces différentes transformations possibles (tel qu'illustré pour le masque de segmentation artificiel 23-6 pour lequel la segmentation de l'anomalie 25 a été à la fois déplacée, réduite, et tournée).

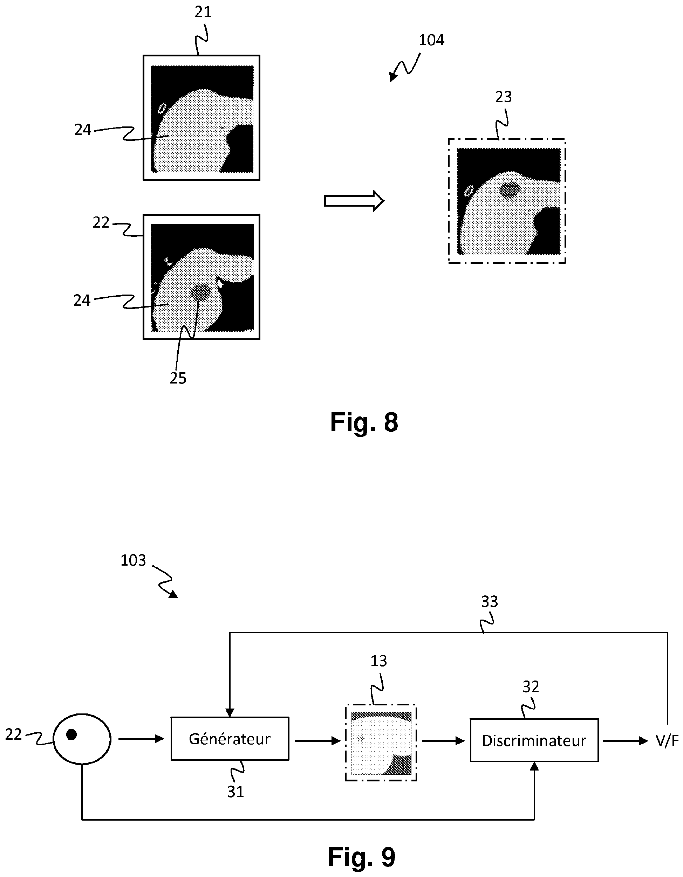

La figure 8 illustre la génération 104 d'un masque de segmentation artificiel 23 à partir d'un masque de segmentation majoritaire 21 et d'un masque de segmentation minoritaire 22. Sur le masque de segmentation majoritaire 21, on peut voir la segmentation de l'anatomie d'intérêt 24-1. Sur le masque de segmentation minoritaire 22, on peut voir à la fois la segmentation de l'anatomie d'intérêt 24-2 et la segmentation de l'anomalie 25-2. Sur le masque de segmentation artificiel 23 généré, on peut voir la segmentation de l'anatomie d'intérêt 24-1 du masque de segmentation majoritaire 21 combinée avec la segmentation de l'anomalie 25-2 du masque de segmentation minoritaire 22 (dans l'exemple considéré, la segmentation de l'anomalie 25-2 a en outre été déplacée).

Dans des modes particuliers de mise en œuvre, la génération 104 d'un masque de segmentation artificiel 23 peut comporter en outre une étape de vérification que la segmentation de l'anomalie 25-2 par rapport à la segmentation de l'anatomie d'intérêt 24-1 satisfait un critère particulier.

Cette étape supplémentaire de vérification permet de s'assurer que certaines contraintes particulières sont satisfaites. Cela peut notamment permettre de filtrer certaines combinaisons non réalistes. Ces contraintes sont par exemple relatives à la localisation de l'anomalie au sein de l'organe d'intérêt ou par rapport à d'autres organes ou d'autres structures anatomiques. Ainsi, à titre d'exemple, le critère peut correspondre au fait qu'une distance entre un bord de la segmentation de l'anatomie d'intérêt et un bord de la segmentation de l'anomalie doit être au moins égale à une valeur seuil. Cela dépend toutefois de l'application visée : si l'on cherche à synthétiser une image de tumeur dans un organe, on fera en sorte que l'anomalie soit située dans l'organe ; si l'on cherche en revanche à synthétiser une image avec des tumeurs dans différents organes, on autorisera la superposition des segmentations des tumeurs avec les segmentations de différents organes.

Cette génération 104 des masques de segmentation artificiels 23, peut être mise en œuvre par un dispositif électronique, comme par exemple un ordinateur. Le dispositif comporte par exemple un ou plusieurs processeurs et au moins un support de stockage lisible par le ou les processeurs. Le support de stockage est destiné à mémoriser les masques de segmentation majoritaires 21 et les masques de segmentation minoritaires 22. Le support de stockage comporte en outre un ensemble d'instructions de code de programme qui, lorsque le programme est exécuté par le ou les processeurs, configurent le ou les processeurs pour générer, tel que décrit ci-avant en référence aux figures 6 à 8, un ensemble de masques de segmentation artificiels 23 à partir des masques de segmentation majoritaires 21 et des masques de segmentation minoritaires 22 mémorisés sur le support de stockage.

La figure 9 représente schématiquement l'étape d'entrainement 103 d'un réseau de neurones 31 à générer une image médicale synthétique 13 à partir d'un masque de segmentation 22.

Dans l'exemple considéré, le réseau de neurones 31 utilisé pour générer une image médicale synthétique 13 est un réseau de neurones générateur 31, et l'entrainement du réseau de neurones générateur 31 est mise en œuvre à l'aide d'un réseau de neurones discriminateur 32. Le réseau de neurones générateur 31 et le réseau de neurones discriminateur 32 formant un couple de réseaux antagonistes génératifs (GAN). Les réseaux de neurones de type GAN permettent en effet de générer des images avec un fort degré de réalisme. Dans un GAN, le réseau de neurones générateur et réseau de neurones discriminateur sont placés en compétition dans un scénario de jeu à somme nulle. Le générateur génère une image, et son adversaire, le discriminateur, essaye de détecter si l'image générée est réelle ou bien s'il s'agit d'une image synthétique générée par le générateur.

Dans un premier temps, le générateur 31 est entrainé à générer des images synthétiques 13 à partir des masques de segmentation. Les masques de segmentation en entrée du générateur 31 peuvent être aussi bien des masques de segmentation minoritaires 22 (comme dans l'exemple illustré à la figure 9) ou des masques de segmentation majoritaires 21.

Dans un deuxième temps, les images synthétiques 13 générées par le générateur sont analysées par le discriminateur 32 qui a été préalablement entrainé à reconnaitre, en prenant en entrée une image 13 et un masque de segmentation 22 associé, si la paire formée par l'image 13 et le masque de segmentation 22 est réelle. Il y a donc en sortie du discriminateur 32 une décision « Vrai » ou « Faux » selon que la paire formée par l'image 13 et le masque de segmentation 22 est considérée comme réelle ou non. Par une boucle de rétropropagation 33, en fonction de la véracité de la décision prise par le discriminateur 32, les paramètres du générateur 31 sont modifiés jusqu'à ce que les images synthétiques 13 générées relatives au masque de segmentation 22 sont considérées comme réelles par le discriminateur 32.

Le générateur 31 est un réseau de neurones de traduction d'une image vers une autre image. Il s'agit par exemple d'un réseau de neurones convolutif de type « pix2pix » tel que décrit dans le document « Image-to-Image translation with conditional adversial networks » par Isola, P et al. Dans l'exemple considéré, le réseau de neurones comporte une première partie de type encodeur composé de couches de convolutions « Batch Normalisation Leaky ReLU » avec des filtres de convolution 4x4 de tailles 64, 128, 256, 512, 512, 512, 512 et une deuxième partie de type décodeur composé de couches de convolution « Batch Normalisation Dropout ReLU » avec des filtres de convolution 4x4 de tailles 512, 512, 512 puis de couches de convolution « Batch Normalisation ReLU » avec des filtres de convolution 4x4 de tailles 256, 128, 64. La réduction de taille d'image dans la partie encodeur est produite par des enjambement de pixels de deux (« strides ») et l'augmentation de la taille de l'image dans la partie décodeur est produite grâce à une couche d'agrandissement 2D (« Upsampling2D », méthode des plus proche voisins ou « Nearest Neighbors ») de taille 2x2. La sortie est produite grâce à une couche d'activation de type tangente hyperbolique (Tanh).

Le discriminateur 32 est un réseau de neurones. Il s'agit par exemple d'un réseau de neurones convolutif de type « PatchGAN » tel que décrit dans le document « Image-to-Image translation with conditional adversial networks » par Isola, P et al, modifié pour accepter deux images en entrée qui sont concaténées en une seule image. Le reste du réseau de neurone est constitué d'une couche de convolution « Leaky ReLU » avec des filtres de convolution 4x4 de taille 64 puis quatre couches de convolution « Batch Normalisation Leaky ReLU » avec des filtres de convolution 4x4 de tailles 128, 256, 512, 512. La sortie est produite à l'aide d'une couche d'activation sigmoïde.

Le réseau de neurones générateur 31 de traduction d'image vers une autre image (« pix2pix ») est combiné avec le discriminateur 32 de type PatchGAN de telle manière que les prédictions de sortie du générateur 31 (les images médicales synthétiques 13) constituent la deuxième entrée du discriminateur 32. La première entrée du discriminateur correspond au masque de segmentation 22 qui est fourni en entrée du générateur 31. La sortie est une matrice de probabilités de 70x70. Les poids des neurones du discriminateur 32 sont non modifiables lors de l'entrainement du générateur 31. Les poids du générateur 31 peuvent être mis à jour lors de l'entrainement. La fonction de calcul du coût est composée de l'entropie croisée et de la norme 1 dans un ratio de 1 pour 100.

Le discriminateur 32 et le générateur 31 sont entrainés en alternance, à tour de rôle, sur un jeu d'entrainement comportant des paires majoritaires (chaque paire majoritaire comporte une image médicale majoritaire et le masque de segmentation majoritaire associé) et des paires minoritaires (chaque paire minoritaire comporte une image médicale minoritaire et le masque de segmentation minoritaire associé). La sortie du discriminateur 32 est optimisée à l'aide de l'algorithme du gradient stochastique de type Adam (Adaptive Moment Estimation, beta_1 :0.9, beta_02 :0.999, epsilon : 1e-08) contre une matrice de 70x70 de valeurs 1 lorsque son entrée est une « vraie » paire (c'est-à-dire une paire comportant un masque de segmentation et son image médicale réelle associée), et de valeur 0 lorsque son entrée est une « fausse » paire (c'est-à-dire une paire comportant un masque de segmentation et une image médicale synthétique produite par le générateur 31 à partir du masque de segmentation). La sortie du générateur 31 est optimisée à l'aide de l'algorithme du gradient stochastique de type Adam contre une matrice de 70x70 de valeurs 1 de manière que les poids des neurones du générateur 31 sont mis à jour, mais pas ceux du discriminateur 32, lorsque le discriminateur 32 détecte que la paire en entrée n'est pas suffisamment proche des « vraies » paires déjà rencontrées. La mise à jour des poids du discriminateur 32 en alternance lui permet d'être en avance sur le générateur 31 et de le forcer à se mettre à jour.

Un réseau de neurones indépendant peut être utilisé pour contrôler l'entraînement des réseaux antagonistes 31, 32. Par exemple, le modèle « InceptionV3 » pré-entrainé peut être utilisé, après avoir retiré la dernière couche de classification, pour comparer les images synthétiques 13 produites par le générateur 31 et les images réelles 12 (« InceptionV3 » est un réseau de neurones convolutif pour aider à l'analyse d'images). Les valeurs d'activations produite en sortie du modèle par les deux images sont utilisées pour calculer un score FID (acronyme pour « Frechet Inception Distance »). Plus ce score est faible, plus les images sont similaires. Les poids du générateur 31 sont sauvegardés dès que le nouveau score FID est inférieur au précédent score enregistré. L'entrainement est arrêté lorsque le score FID devient trop grand par rapport au minimum obtenu au cours de l'entrainement.

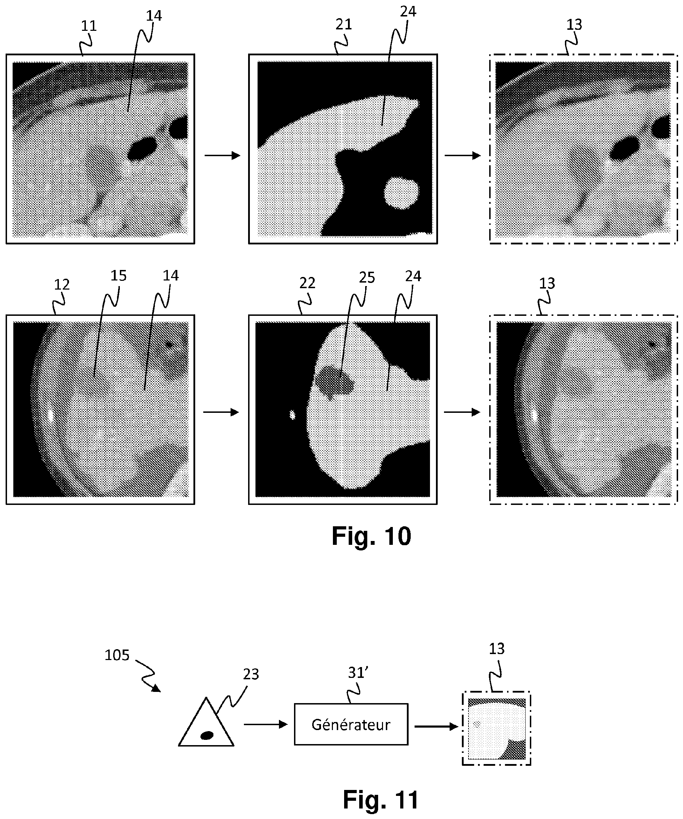

La figure 10 est une illustration d'une image médicale réelle 11, 12, de son masque de segmentation associé 21, 22, et d'une image médicale synthétique 13 générée par le réseau de neurones 31 à partir du masque de segmentation 21, 22, pour un cas majoritaire (sans anomalie) et un cas minoritaire (avec anomalie).

Une fois que l'entrainement du réseau de neurones 31 est terminé, et tel qu'illustré sur la figure 11, le réseau de neurones 31' entrainé peut être utilisé pour générer des images médicales synthétiques 13 à partir des masques de segmentation artificiels 23.

Le dispositif qui met en œuvre l'étape de génération 104 des masques de segmentation artificiels 23 peut également mettre en œuvre l'étape de génération 105 des images médicales synthétiques 13. Dans ce cas, le support de stockage du dispositif mémorise le réseau de neurones générateur 31' préalablement entrainé et, lorsque le programme est exécuté, le ou les processeurs du dispositif sont configurés pour générer des images médicales synthétiques 13 avec le réseau de neurones 31' à partir de masques de segmentation artificiels 23.

La figure 12 illustre à titre d'exemple, pour trois images médicales réelles majoritaires 11 différentes : l'image médicale réelle majoritaire 11, son masque de segmentation majoritaire 21 associé, un masque de segmentation artificiel 23 généré à partir du masque de segmentation majoritaire, et une image médicale synthétique 13 générée par le réseau de neurones 31' à partir du masque de segmentation artificiel 23.

Grâce à la grande diversité de masques de segmentation artificiels 23 générés à l'étape 104, il est possible de générer une grande diversité d'images médicales synthétiques 13 présentant des anomalies. Cette diversité des images médicales synthétiques 13 permet de générer un jeu d'images d'entrainement particulièrement efficace pour un algorithme d'apprentissage automatique visant à détecter ou caractériser une anomalie dans l'anatomie d'intérêt d'un patient sur une image médicale réelle. Le jeu d'images d'entrainement peut comprendre à la fois des images médicales réelles et des images médicales synthétiques. De préférence, le jeu d'images d'entrainement comprend un nombre d'images avec anomalie sensiblement égal au nombre d'images sans anomalie. Cela permet ainsi d'éviter le phénomène de sur-apprentissage sur une classe particulière d'anomalie.

La figure 13 représente schématiquement l'étape d'entrainement 202, à partir d'une image médicale synthétique 13, de l'algorithme d'apprentissage automatique 40. L'algorithme d'apprentissage automatique 40 vise à détecter ou caractériser une anomalie dans l'anatomie d'intérêt d'un patient sur une image médicale. Dans l'exemple considéré, l'algorithme d'apprentissage automatique 40 est un réseau de neurones profonds.

Tel qu'illustré sur la figure 13, pendant la phase d'entrainement, l'algorithme d'apprentissage automatique 40 prend en entrée une image médicale (il s'agit d'une image médicale synthétique 13 sur l'exemple illustré à la figure 13). L'information qui doit être obtenue en sortie de l'algorithme d'apprentissage automatique 40 est connue a priori (« information attendue »). L'information obtenue et l'information attendue sont comparée et, en fonction du résultat de la comparaison, les paramètres du réseau de neurones sont mis à jour par une boucle de rétropropagation 41. L'entrainement est poursuivi jusqu'à ce que l'algorithme d'apprentissage automatique 40 est capable de fournir l'information attendue avec un taux de réussite satisfaisant.

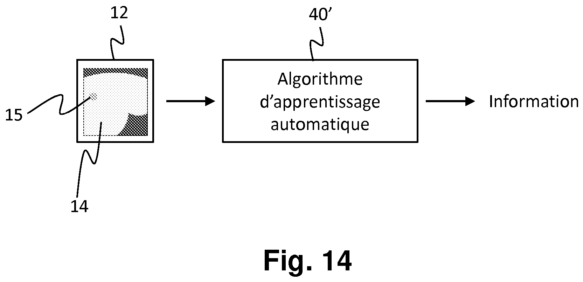

La figure 14 représente schématiquement la détection ou la caractérisation d'une anomalie 15 dans une anatomie d'intérêt 14 représentée sur une image médicale réelle 12 par l'algorithme d'apprentissage automatique 40' ainsi entrainé. L'information fournie en sortie de l'algorithme d'apprentissage automatique 40' correspond par exemple à une indication qu'une anomalie a été détectée, à une classification de l'anomalie (nature de la tumeur par exemple) et/ou à une segmentation de l'anomalie sur l'image médicale 12.

Dans un mode de fonctionnement alternatif, l'entraînement du réseau de neurones 31 est concomitant à l'entraînement de l'algorithme d'apprentissage automatique 40. Dans ce mode de fonctionnement, l'entraînement du réseau de neurones 31 n'est pas arrêté et les images synthétiques sont produites par le réseau de neurones 31 et utilisées par l'algorithme d'apprentissage automatique 40 à intervalles réguliers sans étape de stockage intermédiaire. Chaque lot d'images synthétiques est produit par le réseau de neurones 31 à différentes étapes d'entrainement, par conséquent un même masque de segmentation artificiel 23 produira une image synthétique différente. Ce fonctionnement permet une variation infinie des images produites par le réseau de neurones 31 à destination de l'algorithme d'apprentissage automatique 40. Cette caractéristique est importante pour limiter le sur-apprentissage de l'algorithme d'apprentissage automatique 40 des données d'entraînement puisque l'algorithme n'utilisera jamais deux fois la même image synthétique au cours de l'entraînement.

## Revendications

1. Méthode (100) pour générer des images médicales synthétiques (13) représentant une anatomie d'intérêt (14) et une anomalie (15) au sein de ladite anatomie d'intérêt (14), ladite méthode (100) comprenant :
- une génération (101) de masques de segmentation majoritaires (21), chaque masque de segmentation majoritaire (21) étant associé à une image médicale réelle majoritaire (11) représentant l'anatomie d'intérêt (14) d'un patient sans anomalie,
- une génération (102) de masques de segmentation minoritaires (22), chaque masque de segmentation minoritaire (22) étant associé à une image médicale réelle minoritaire (12) représentant l'anatomie d'intérêt (14) d'un patient avec une anomalie (15),
- un entrainement (103) d'un réseau de neurones (31) pour générer une image médicale synthétique (13) à partir d'un masque de segmentation,
- une génération (104) de masques de segmentation artificiels (23) à partir des masques de segmentation majoritaires (21) et des masques de segmentation minoritaires (22), la génération (104) d'un masque de segmentation artificiel (23) comportant une combinaison d'une segmentation de l'anatomie d'intérêt (24) d'un masque de segmentation majoritaire (21) avec une segmentation de l'anomalie (25) d'un masque de segmentation minoritaire (22),
- une sélection de masques de segmentation artificiels (23) en fonction d'une localisation de l'anomalie au sein de l'organe d'intérêt ou par rapport à d'autres organes ou d'autres structures anatomiques,
- une génération (105) d'images médicales synthétiques (13), à partir des masques de segmentation artificiels (23) à l'aide du réseau de neurones (31') préalablement entrainé.

2. Méthode (100) selon la revendication 1 dans laquelle la génération (104) d'un masque de segmentation artificiel (23-1 à 23-7) comporte en outre une transformation de la segmentation de l'anomalie (25) du masque de segmentation minoritaire (22).

3. Méthode selon la revendication 2 dans laquelle la transformation de la segmentation de l'anomalie (25) correspond à une rotation, un agrandissement, une réduction, une déformation et/ou un déplacement de la segmentation de l'anomalie (25).

4. Méthode (100) selon l'une quelconque des revendications 1 à 3 dans laquelle un masque de segmentation (21, 22) comporte un ensemble de voxels, chaque voxel correspondant à une zone de l'image médicale réelle (11, 12) à laquelle le masque de segmentation (21, 22) est associé, chaque voxel étant associé à une valeur numérique encodant ce qui est représenté par ladite zone sur l'image médicale réelle (11, 12), et l'étape de génération (104) d'un masque de segmentation artificiel (23) comporte :
- une sélection d'un masque de segmentation majoritaire (21) et d'un masque de segmentation minoritaire (22),
- une identification, sur le masque de segmentation minoritaire (22) sélectionné, d'un ensemble de voxels dont la valeur numérique encode l'anomalie,
- un remplacement, sur le masque de segmentation majoritaire (21) sélectionné, de la valeur numérique des voxels identifiés par la valeur numérique encodant l'anomalie.

5. Méthode (100) selon l'une quelconque des revendications 1 à 4 dans laquelle le réseau de neurones (31) utilisé pour générer une image médicale synthétique (13) est un réseau de neurones générateur (31), et l'entrainement du réseau de neurones générateur (31) est mise en œuvre à l'aide d'un réseau de neurones discriminateur (32), le réseau de neurones générateur (31) et le réseau de neurones discriminateur (32) formant un couple de réseaux antagonistes génératifs.

6. Méthode (100) selon l'une quelconque des revendications 1 à 5 dans laquelle les images médicales réelles (11, 12) à partir desquelles sont générés les masques de segmentation majoritaires (21) et les masques de segmentation minoritaires (22) sont des images médicales obtenues par tomodensitométrie, par tomographie par émission de positons par imagerie par résonance magnétique ou par ultrasons.

7. Méthode (100) selon l'une quelconque des revendications 1 à 6 dans laquelle l'anatomie d'intérêt est un organe tel que le foie, un poumon ou un rein, ou une autre structure anatomique telle qu'un os ou un vaisseau sanguin.

8. Méthode (100) selon l'une quelconque des revendications 1 à 7 dans laquelle l'anomalie est une tumeur ou une zone d'ablation.

9. Méthode (200) pour entrainer un algorithme d'apprentissage automatique (40) visant à détecter ou caractériser une anomalie (15) dans l'anatomie d'intérêt (14) d'un patient sur une image médicale réelle (11, 12), ladite méthode (200) comprenant :
- une génération (201), avec une méthode (100) selon l'une quelconque des revendications 1 à 8, d'images médicales synthétiques (13) représentant l'anatomie d'intérêt et une anomalie au sein de ladite anatomie intérêt,
- un entrainement (202) de l'algorithme d'apprentissage automatique (40) avec un ensemble d'images d'entrainement comportant les images médicales synthétiques (13) ainsi générées.

10. Méthode (200) selon la revendication 9 dans laquelle l'ensemble d'images d'entrainement comporte des images médicales synthétiques (13) et des images médicales réelles (11, 12), et le nombre d'images avec une anomalie est au moins égal à 10% du nombre d'images sans anomalie.

11. Méthode (200) selon l'une quelconque des revendications 9 à 10 dans laquelle l'algorithme d'apprentissage automatique (40) est un algorithme de classification de l'anomalie.

12. Méthode (200) selon l'une quelconque des revendications 9 à 10 dans laquelle l'algorithme d'apprentissage automatique (40) est un algorithme de segmentation de l'anomalie.

13. Méthode (200) selon l'une quelconque des revendications 9 à 12 dans laquelle l'algorithme d'apprentissage automatique (40) est un mis en œuvre par un réseau de neurones profonds.

14. Dispositif comportant un ou plusieurs processeurs et au moins un support de stockage lisible par le ou les processeurs, le support de stockage étant destiné à mémoriser des masques de segmentation majoritaires (21) et des masques de segmentation minoritaires (22), chaque masque de segmentation majoritaire (21) comportant une segmentation d'une anatomie d'intérêt (24) visible sur une image médicale réelle majoritaire (11) de l'anatomie d'intérêt (14) d'un patient sans anomalie, chaque masque de segmentation minoritaire (22) comportant une segmentation d'une anomalie (25) visible sur une image médicale réelle minoritaire (12) de l'anatomie d'intérêt (14) d'un patient avec une anomalie (15) au sein de ladite anatomie d'intérêt (14), le dispositif étant **caractérisé en ce que** le support de stockage comporte un ensemble d'instructions de code de programme qui, lorsque le programme est exécuté par le ou les processeurs, configurent le ou les processeurs pour mettre en œuvre la méthode selon la revendication 1.

15. Dispositif selon la revendication 14 dans lequel, pour générer un masque de segmentation artificiel (23-1 à 23-7), le ou les processeurs sont en outre configurés pour transformer la segmentation de l'anomalie (25) du masque de segmentation minoritaire (22).

16. Dispositif selon la revendication 15 dans lequel la transformation de la segmentation de l'anomalie (25) correspond à une rotation, un agrandissement, une réduction, une déformation ou un déplacement de la segmentation de l'anomalie (25).

17. Dispositif selon l'une quelconque des revendications 14 à 16 dans laquelle un masque de segmentation (21, 22) comporte un ensemble de voxels, chaque voxel correspondant à une zone de l'image médicale réelle (11, 12) à laquelle le masque de segmentation (21, 22) est associé, chaque voxel étant associé à une valeur numérique encodant ce qui est représenté par ladite zone sur l'image médicale réelle (11, 12) et, pour générer un masque de segmentation artificiel (23), le ou les processeurs sont configurés pour :
- sélectionner un masque de segmentation majoritaire (21) et un masque de segmentation minoritaire (22),
- identifier, sur le masque de segmentation minoritaire (22) sélectionné, un ensemble de voxels dont la valeur numérique encode l'anomalie,
- remplacer, sur le masque de segmentation majoritaire (21) sélectionné, la valeur numérique des voxels identifiés par la valeur numérique encodant l'anomalie.

18. Dispositif selon la revendication 14-17 dans lequel le réseau de neurones (31) pour générer une image médicale synthétique (13) est un réseau de neurones générateur (31) adapté pour être entrainé à l'aide d'un réseau de neurones discriminateur (32), le réseau de neurones générateur (31) et le réseau de neurones discriminateur (32) formant un couple de réseaux antagonistes génératifs.

## Patentansprüche

1. Verfahren (100) zum Erzeugen synthetischer medizinischer Bilder (13), die eine Anatomie von Interesse (14) und eine Anomalie (15) innerhalb der Anatomie von Interesse (14) darstellen, wobei das Verfahren (100) Folgendes umfasst:
- Erzeugen (101) von Mehrheits-Segmentierungsmasken (21), wobei jede Mehrheits-Segmentierungsmaske (21) einem tatsächlichen medizinischen Mehrheits-Bild (11) zugeordnet ist, das die Anatomie von Interesse (14) eines Patienten ohne Anomalien darstellt,
- Erzeugen (102) von Minderheits-Segmentierungsmasken (22), wobei jede Minderheits-Segmentierungsmaske (22) einem tatsächlichen medizinischen Minderheits-Bild (12) zugeordnet ist, das die Anatomie von Interesse (14) eines Patienten mit einer Anomalie (15) darstellt,
- Trainieren (103) eines neuronalen Netzwerks (31) zum Erzeugen eines synthetischen medizinischen Bildes (13) aus einer Segmentierungsmaske,
- Erzeugen (104) künstlicher Segmentierungsmasken (23) aus den Mehrheits-Segmentierungsmasken (21) und den Minderheits-Segmentierungsmasken (22), wobei das Erzeugen (104) einer künstlichen Segmentierungsmaske (23) eine Kombination einer Segmentierung der Anatomie von Interesse (24) einer Mehrheits-Segmentierungsmaske (21) mit einer Segmentierung der Anomalie (25) einer Minderheits-Segmentierungsmaske (22) aufweist,
- Auswählen künstlicher Segmentierungsmasken (23) in Abhängigkeit von einer Lokalisierung der Anomalie innerhalb des Organs von Interesse oder in Bezug auf andere Organe oder andere anatomische Strukturen,
- Erzeugen (105) synthetischer medizinischer Bilder (13) aus den künstlichen Segmentierungsmasken (23) mithilfe des zuvor trainierten neuronalen Netzwerks (31').

2. Verfahren (100) nach Anspruch 1, wobei das Erzeugen (104) einer künstlichen Segmentierungsmaske (23-1 bis 23-7) ferner eine Transformation der Segmentierung der Anomalie (25) der Minderheits-Segmentierungsmaske (22) aufweist.

3. Verfahren nach Anspruch 2, wobei die Transformation der Segmentierung der Anomalie (25) einer Drehung, Vergrößerung, Reduzierung, Verformung und/oder Verschiebung der Segmentierung der Anomalie (25) entspricht.

4. Verfahren (100) nach einem der Ansprüche 1 bis 3, wobei eine Segmentierungsmaske (21, 22) einen Satz von Voxeln aufweist, wobei jedes Voxel einem Bereich des tatsächlichen medizinischen Bildes (11, 12) entspricht, dem die Segmentierungsmaske (21, 22) zugeordnet ist, wobei jedem Voxel ein numerischer Wert zugeordnet ist, der das kodiert, was durch den Bereich auf dem tatsächlichen medizinischen Bild (11, 12) dargestellt wird, und der Schritt (104) des Erzeugens einer künstlichen Segmentierungsmaske (23) Folgendes aufweist:
- Auswählen einer Mehrheits-Segmentierungsmaske (21) und einer Minderheits-Segmentierungsmaske (22),
- Identifizieren, auf der ausgewählten Minderheits-Segmentierungsmaske (22), eines Satzes von Voxeln, dessen numerischer Wert die Anomalie kodiert,
- Ersetzen, auf der ausgewählten Mehrheits-Segmentierungsmaske (21), des numerischen Werts der Voxel, die durch den numerischen Wert identifiziert wurden, der die Anomalie kodiert.

5. Verfahren (100) nach einem der Ansprüche 1 bis 4, wobei das zum Erzeugen eines synthetischen medizinischen Bildes (13) verwendete neuronale Netzwerk (31) ein generierendes neuronales Netzwerk (31) ist und das Training des generierenden neuronalen Netzwerks (31) mithilfe eines diskriminierenden neuronalen Netzwerks (32) durchgeführt wird, wobei das generierende neuronale Netzwerk (31) und das diskriminierende neuronale Netzwerk (32) ein Paar generativer antagonistischer Netzwerke bilden.

6. Verfahren (100) nach einem der Ansprüche 1 bis 5, wobei die tatsächlichen medizinischen Bilder (11, 12), aus denen die Mehrheits-Segmentierungsmasken (21) und die Minderheits-Segmentierungsmasken (22) erzeugt werden, medizinische Bilder sind, die durch Computertomographie, Positronen-Emissions-Tomographie, Magnetresonanz-Bildgebung oder Ultraschall gewonnen wurden.

7. Verfahren (100) nach einem der Ansprüche 1 bis 6, wobei die Anatomie von Interesse ein Organ wie Leber, Lunge oder Niere oder eine andere anatomische Struktur wie Knochen oder Blutgefäß ist.

8. Verfahren (100) nach einem der Ansprüche 1 bis 7, wobei die Anomalie ein Tumor oder ein Ablationsbereich ist.

9. Verfahren (200) zum Trainieren eines maschinellen Lernalgorithmus (40), der darauf abzielt, eine Anomalie (15) in der Anatomie von Interesse (14) eines Patienten auf einem tatsächlichen medizinischen Bild (11, 12) zu erkennen oder zu charakterisieren, wobei das Verfahren (200) Folgendes umfasst:
- Erzeugen (201), mit einem Verfahren (100) nach einem der Ansprüche 1 bis 8, von synthetischen medizinischen Bildern (13), die die Anatomie von Interesse und eine Anomalie innerhalb der Anatomie von Interesse darstellen,
- Trainieren (202) des maschinellen Lernalgorithmus (40) mit einem Satz von Trainingsbildern, die die so erzeugten synthetischen medizinischen Bilder (13) aufweisen.

10. Verfahren (200) nach Anspruch 9, wobei der Satz von Trainingsbildern synthetische medizinische Bilder (13) und tatsächliche medizinische Bilder (11, 12) aufweist und die Anzahl der Bilder mit einer Anomalie mindestens 10 % der Anzahl der Bilder ohne Anomalie entspricht.

11. Verfahren (200) nach einem der Ansprüche 9 bis 10, wobei der maschinelle Lernalgorithmus (40) ein Algorithmus zur Klassifizierung der Anomalie ist.

12. Verfahren (200) nach einem der Ansprüche 9 bis 10, wobei der maschinelle Lernalgorithmus (40) ein Algorithmus zur Segmentierung der Anomalie ist.

13. Verfahren (200) nach einem der Ansprüche 9 bis 12, wobei der maschinelle Lernalgorithmus (40) einer durch ein tiefes neuronales Netzwerk implementierter ist.

14. Vorrichtung, die einen oder mehrere Prozessoren und mindestens einen von dem oder den Prozessoren lesbaren Speichermedium aufweist, wobei das Speichermedium dazu bestimmt ist, Mehrheits-Segmentierungsmasken (21) und Minderheits-Segmentierungsmasken (22) zu speichern, wobei jede Mehrheits-Segmentierungsmaske (21) eine Segmentierung einer Anatomie von Interesse (24) aufweist, die auf einem tatsächlichen medizinischen Mehrheits-Bild (11) der Anatomie von Interesse (14) eines Patienten ohne Anomalien sichtbar ist, wobei jede Minderheits-Segmentierungsmaske (22) eine Segmentierung einer Anomalie (25) aufweist, die auf einem tatsächlichen medizinischen MinderheitsBild (12) der Anatomie von Interesse (14) eines Patienten mit einer Anomalie (15) innerhalb der Anatomie von Interesse (14) sichtbar ist, wobei die Vorrichtung **dadurch gekennzeichnet ist, dass** das Speichermedium eine Reihe von Programmcodeanweisungen aufweist, die, wenn das Programm von dem oder den Prozessoren ausgeführt wird, den beziehungsweise die Prozessoren so konfigurieren, dass sie das Verfahren nach Anspruch 1 implementieren.

15. Vorrichtung nach Anspruch 14, wobei der oder die Prozessoren zum Erzeugen einer künstlichen Segmentierungsmaske (23-1 bis 23-7) ferner so konfiguriert sind, dass sie die Segmentierung der Anomalie (25) der Minderheits-Segmentierungsmaske (22) transformieren.

16. Vorrichtung nach Anspruch 15, wobei die Transformation der Segmentierung der Anomalie (25) einer Drehung, Vergrößerung, Verkleinerung, Verformung oder Verschiebung der Segmentierung der Anomalie (25) entspricht.

17. Vorrichtung nach einem der Ansprüche 14 bis 16, wobei eine Segmentierungsmaske (21, 22) einen Satz von Voxeln aufweist, wobei jedes Voxel einem Bereich des tatsächlichen medizinischen Bildes (11, 12) entspricht, dem die Segmentierungsmaske (21, 22) zugeordnet ist, wobei jedem Voxel ein numerischer Wert zugeordnet ist, der kodiert, was durch den Bereich auf dem tatsächlichen medizinischen Bild (11, 12) dargestellt wird, und der beziehungsweise die Prozessoren zum Erzeugen einer künstlichen Segmentierungsmaske (23) für Folgendes konfiguriert sind:
- Auswählen einer Mehrheits-Segmentierungsmaske (21) und einer Minderheits-Segmentierungsmaske (22),
- Identifizieren, auf der ausgewählten Minderheits-Segmentierungsmaske (22), eines Satzes von Voxeln, dessen numerischer Wert die Anomalie kodiert,
- Ersetzen, auf der ausgewählten Mehrheits-Segmentierungsmaske (21), des numerischen Werts der Voxel, die durch den numerischen Wert identifiziert wurden, der die Anomalie kodiert.

18. Vorrichtung nach den Ansprüchen 14-17, wobei das neuronalen Netzwerk (31) zum Erzeugen eines synthetischen medizinischen Bildes (13) ein generierendes neuronales Netzwerk (31) ist, das geeignet ist, mithilfe eines diskriminierenden neuronalen Netzwerks (32) trainiert zu werden, wobei das generierende neuronale Netzwerk (31) und das diskriminierende neuronale Netzwerk (32) ein Paar generativer antagonistischer Netzwerke bilden.

## Claims

1. Method (100) for generating synthetic medical images (13) representing an anatomy of interest (14) and an anomaly (15) within said anatomy of interest (14), said method (100) including:
- generating(101) majority segmentation masks (21), each majority segmentation mask (21) being associated with a majority real medical image (11) representing the anatomy of interest (14) of a patient without abnormality,
- generating(102) minority segmentation masks (22), each minority segmentation mask (22) being associated with a minority real medical image (12) representing the anatomy of interest (14) of a patient with an anomaly (15),
- training (103) a neural network (31) to generate a synthetic medical image (13) on the basis of a segmentation mask,
- generating (104) artificial segmentation masks (23) on the basis of majority segmentation masks (21) and minority segmentation masks (22), generating (104) an artificial segmentation mask (23) comprising combining a segmentation of the anatomy of interest (24) of a majority segmentation mask (21) with a segmentation of the anomaly (25) of a minority segmentation mask (22),
- selecting artificial segmentation masks (23) depending on a location of the anomaly within the organ of interest or in relation to other organs or other anatomical structures,
- generating (105) synthetic medical images (13), on the basis of the artificial segmentation masks (23) using the previously trained neural network (31').

2. Method (100) according to claim 1, wherein the generation (104) of an artificial segmentation mask (23-1 to 23-7) further comprises transforming the segmentation of the anomaly (25) of the minority segmentation mask (22).

3. Method according to claim 2 wherein transforming the segmentation of the anomaly (25) corresponds to rotating, magnifying, reducing, deforming and/or moving the segmentation of the anomaly (25).

4. Method (100) according to any one of claims 1 to 3 wherein a segmentation mask (21, 22) comprises a set of voxels, each voxel corresponding to an area of the real medical image (11, 12) to which the segmentation mask (21, 22) is associated, each voxel being associated with a numerical value encoding what is represented by said area on the real medical image (11, 12), and the step of generating (104) an artificial segmentation mask (23) comprises:
- selecting a majority segmentation mask (21) and a minority segmentation mask (22),
- identifying, on the selected minority segmentation mask (22), a set of voxels the numerical value of which encodes the anomaly,
- replacing, on the selected majority segmentation mask (21), the numerical value of the voxels identified by the numerical value encoding the anomaly.

5. Method (100) according to any one of claims 1 to 4 wherein the neural network (31) used to generate a synthetic medical image (13) is a generator neural network (31), and training the generator neural network (31) is implemented using a discriminator neural network (32), the generator neural network (31) and the discriminator neural network (32) forming a pair of generative antagonist networks.

6. Method (100) according to any one of claims 1 to 5 wherein the real medical images (11, 12) on the basis of which the majority segmentation masks (21) and the minority segmentation masks (22) are generated are medical images obtained by computed tomography, by positron emission tomography by magnetic resonance imaging or by ultrasound.

7. Method (100) according to any one of claims 1 to 6, wherein the anatomy of interest is an organ such as the liver, a lung or a kidney, or another anatomical structure such as a bone or a blood vessel.

8. Method (100) according to any one of claims 1 to 7 wherein the anomaly is a tumor or an ablation zone.

9. Method (200) for training a machine learning algorithm (40) for detecting or characterizing an anomaly (15) in the anatomy of interest (14) of a patient on a real medical image (11, 12), said method (200) including:
- generating (201), with a method (100) according to any one of claims 1 to 8, synthetic medical images (13) representing the anatomy of interest and an anomaly within said anatomy of interest,
- training (202) the machine learning algorithm (40) with a set of training images comprising the synthetic medical images (13) thus generated.

10. Method (200) according to claim 9, wherein the set of training images comprises synthetic medical images (13) and real medical images (11, 12), and the number of images with an anomaly is at least equal to 10% of the number of images without an anomaly.

11. Method (200) according to any one of claims 9 to 10 wherein the machine learning algorithm (40) is an algorithm for classifying the anomaly.

12. Method (200) according to any one of claims 9 to 10 wherein the machine learning algorithm (40) is an algorithm for segmenting the anomaly.

13. Method (200) according to any one of claims 9 to 12 wherein the machine learning algorithm (40) is implemented by a deep neural network.

14. Device comprising one or more processors and at least one storage medium readable by the processor(s), the storage medium being intended to memorize majority segmentation masks (21) and minority segmentation masks (22), each majority segmentation mask (21) comprising segmenting an anatomy of interest (24) visible on a majority real medical image (11) of the anatomy of interest (14) of a patient without abnormality, each minority segmentation mask (22) comprising segmenting an anomaly (25) visible on a minority real medical image (12) of the anatomy of interest (14) of a patient with an anomaly (15) within said anatomy of interest (14), the device being **characterized in that** the storage medium comprises a set of program code instructions which, when the program is executed by the processor(s), configure the processor(s) to implement the method according to claim 1.

15. Device according to claim 14 wherein, to generate an artificial segmentation mask (23-1 to 23-7), the processor(s) is/are further configured to transform the segmentation of the anomaly (25) of the minority segmentation mask (22).

16. Device according to claim 15, wherein transforming the segmentation of the anomaly (25) corresponds to rotating, magnifying, reducing, deforming or moving the segmentation of the anomaly (25).

17. Device according to any one of claims 14 to 16, wherein a segmentation mask (21, 22) comprises a set of voxels, each voxel corresponding to an area of the real medical image (11, 12) to which the segmentation mask (21, 22) is associated, each voxel being associated with a numerical value encoding what is represented by said area on the real medical image (11, 12) and, to generate an artificial segmentation mask (23), the processor(s) is/are configured to:
- select a majority segmentation mask (21) and a minority segmentation mask (22),
- identify on the selected minority segmentation mask (22), a set of voxels the numerical value of which encodes the anomaly,
- replace, on the selected majority segmentation mask (21), the numerical value of the voxels identified by the numerical value encoding the anomaly.

18. Device according to claims 14-17 wherein the neural network (31) for generating a synthetic medical image (13) is a generator neural network (31) adapted to be trained using a discriminator neural network (32), the generator neural network (31) and the discriminator neural network (32) forming a pair of generative antagonist networks.
